# EUROPÄISCHE PATENTSCHRIFT

(11) **EP 1 922 958 B1**
(45) Veröffentlichungstag und Bekanntmachung des Hinweises auf die Patenterteilung: **13.03.2013**
(21) Anmeldenummer: 06023793.0
(22) Anmeldetag: 16.11.2006
(51) Int. Cl.: A47F 3/04

(54) **Aggregat aus Warenpräsentationsmöbel und Kühleinrichtung**
Unit comprising a show case and a refrigerating device
Ensemble constitué d'un meuble de présentation et un dispositif réfrigérant

(43) Veröffentlichungstag der Anmeldung: 21.05.2008
(73) Patentinhaber: Aldi Einkauf GmbH & Co. oHG, 45476 Mülheim/Ruhr (DE)
(72) Erfinder: Ochsenschläger, Robert Kaufmann, 45470 Mülheim/Ruhr (DE); Ernst, Peter Kaufmann, 45478 Mülheim/Ruhr (DE); Bader Thomas, 97080 Würzburg (DE)
(74) Vertreter: Nunnenkamp, Jörg

(56) Entgegenhaltungen:
- EP-A- 1 600 084
- CA-A1- 2 412 244
- DE-A1- 2 646 380
- DE-A1- 10 307 301
- FR-A1- 2 715 816
- US-A- 2 915 884
- US-A- 3 392 543

## Beschreibung

Die Erfindung betrifft ein Aggregat aus Warenpräsentationsmöbel und hiermit verbundener Kühleinrichtung, wobei die Kühleinrichtung kopfseitig mit einer Luftführungseinheit ausgerüstet und zusätzlich eine Rücklufteinrichtung vorgesehen ist, wobei sich ferner das Warenpräsentationsmöbel aus einem Gestell und einer Warenauflage zusammensetzt sowie die Warenauflage gegenüber der im Wesentlichen als senkrechter Turm ausgebildeten Kühleinrichtung schräggestellt ist, und wobei die Luftführungseinheit die Warenauflage des Warenpräsentationsmöbels überragt, so dass die aus der Luftführungseinheit austretende Kühlluft in etwa parallel über dort platzierte Waren zu deren Temperierung streicht.

Ein derartiges Aggregat bzw. eine Einheit aus einem Warenpräsentationsmöbel und einer hiermit verbundenen Kühleinrichtung wird in der CA 2 412 244 A1 beschrieben. Hier geht es um ein sanitäres System, welches einen Luftschleier erzeugt und mit einem Desinfektionsmittel beaufschlagt. Dadurch soll etwaiger Bakterienbefall bei Früchten und Gemüse vermieden werden, wenn diese mit einem Wassernebel befeuchtet werden.

Andere Aggregate aus Warenpräsentationsmöbel und Kühleinrichtung sind aus der Praxis vielfältig bekannt und werden beispielhaft in dem Gebrauchsmuster DE 296 13 775 U1 beschrieben. Ähnlich gehen die DE 31 13 570 A1, die DE 298 08 428 U1 oder auch die EP 1 627 584 A1 vor. Das hat sich bewährt.

Im Rahmen der DE 103 07 301 A1 wird ein neuartiges Warenpräsentationsmöbel beschrieben, bei welchem eine Warenpräsentationseinheit und eine Kühleinheit relativ zueinander verfahr- und/oder verschiebbar angeordnet sind. Dadurch soll insbesondere die Wartung sowie Reinigung vorhandener Komponenten verbessert werden.

Im Stand der Technik formen das Aggregat aus Warenpräsentationsmöbel und Kühleinrichtung durchweg ein kombiniertes Kühlregal oder ein Kühlmöbel. Dabei besteht zwar die Möglichkeit, eine oder mehrere Warenauflageebenen zu verstellen, um unterschiedliche Waren, Warenpräsentationen etc. Rechnung zu tragen. In der Praxis werden jedoch zunehmend Anforderungen dahingehend gestellt, eine noch größere Flexibilität zu ermöglichen. Das gilt nicht nur im Hinblick auf die Verstellung der Warenauflagen, sondern ganz allgemein unter Berücksichtigung des Aufstellungsortes, der gewünschten Warenpräsentation, der örtlichen Gegebenheiten im Ladengeschäft etc.. Hier setzt die Erfindung ein.

Der Erfindung liegt das technische Problem zugrunde, ein derartiges Aggregat so weiterzuentwickeln, dass eine größtmögliche Flexibilität erreicht wird. Außerdem sollen die Kosten verringert werden.

Zur Lösung dieser technischen Problemstellung schlägt die Erfindung bei einem gattungsgemäßen Aggregat aus Warenpräsentationsmöbel und Kühleinrichtung vor, dass das Warenpräsentationsmöbel und die hiermit verbundene Kühleinrichtung jeweils modular mit zumindest eigenem Gestell ausgebildet sind, wobei zusätzlich die Rücklufteinrichtung ebenfalls selbst modular mit zumindest einem Gestell ausgeführt ist. - Nach vorteilhafter Ausgestaltung können das Warenpräsentationsmöbel und die Kühleinrichtung auch mit einem Gehäuse ausgerüstet werden. Außerdem verfügt die Rücklufteinrichtung vorteilhaft über ein Gehäuse und ist optional verfahrbar ausgebildet.

Bei dem Warenpräsentationsmöbel handelt es sich um übliche Warenpräsentationsregale, Warenpräsentationsträger oder dergleichen. Diese können als Gittergestelle, Tischgestelle, Regalgestelle etc. ausgeführt sein. Dabei kann selbstverständlich auch auf bereits vorhandene Warenpräsentationsmöbel zurückgegriffen werden.

Entscheidend ist die Tatsache, dass erfindungsgemäß das Warenpräsentationsmöbel und die Kühleinrichtung unabhängig voneinander und gleichsam modular gestaltet sind. Das wird durch das jeweils zwingende eigene Gestell erreicht, so dass sowohl das Warenpräsentationsmöbel als auch die Kühleinrichtung eine jeweils selbsttragende Struktur formen. Wie bereits erläutert, mögen das Warenpräsentationsmöbel und die Kühleinrichtung auch mit einem Gehäuse ausgerüstet werden, was jedoch nicht zwingend ist.

In der Regel handelt es sich bei dem Warenpräsentationsmöbel um einen Warenpräsentationstisch, welcher oftmals über eine im Vergleich zu einer Basis demgegenüber verstellbare Warenauflage verfügt. Solche Warenpräsentationstische werden allgemein auch als Verkaufsgestell bezeichnet und sind beispielsweise Gegenstand der EP 1 493 363 A1, auf die in diesem Zusammenhang ausdrücklich verwiesen sei.

Das Warenpräsentationsmöbel bzw. der an dieser Stelle zumeist realisierte Warenpräsentationstisch mit oberseitiger und ggf. verstellbarer Warenauflage lässt sich nun problemlos mit Hilfe der Kühleinrichtung beaufschlagen. Denn die Kühleinrichtung ist kopfseitig mit der Luftführungseinheit ausgerüstet, bei der es sich beispielhaft um ein Lamellengitter handeln kann. Dabei ist die Auslegung so getroffen, dass die Luftführungseinheit der Kühleinrichtung eine (oberste) Warenauflage bzw. Warenauflageebene des Warenpräsentationsmöbels überragt. Auf diese Weise kann die aus der Kühleinrichtung über die Luftführungseinheit austretende Kühlluft problemlos über die auf der Warenauflage zumeist platzierten Waren streichen.

Die Warenauflage bzw. Warenauflageebene ist schräg abfallend angeordnet, und zwar ausgehend von der Kühleinrichtung, welche randseitig mit dem Warenpräsentationsmöbel bzw. dem Warenpräsentationstisch verbunden ist. Um dies im Detail zu erreichen, sorgt die Luftführungseinheit dafür, dass die austretende Kühlluft der Schrägstellung der Warenauflage folgend geführt wird. Zu diesem Zweck sind die an dieser Stelle zumeist vorgesehenen Lamellen des Lamellengitters entsprechend geneigt. Dabei entspricht der Neigungswinkel der Lamellen im Wesentlichen dem Neigungswinkel der Warenauflage bzw. der Warenauflageebene.

Die ergänzend zu dem Warenpräsentationsmöbel und der Kühleinrichtung vorgesehene Rücklufteinrichtung könnte grundsätzlich Bestandteil des Warenpräsentationsmöbels sein. Erfindungsgemäß ist die Rücklufteinrichtung jedoch selbst modular aufgebaut und verfügt zu diesem Zweck über ein eigenes Gestell und ggf. ein Gehäuse. Damit die Rücklufteinrichtung die aus der Kühleinrichtung bzw. der kopfseitig vorgesehenen Luftführungseinheit austretende Kühlluft insgesamt im Kreislauf zur Kühleinrichtung zurückführen kann, ist die Rücklufteinrichtung regelmäßig an einer der Kühleinrichtung gegenüberliegenden Seite des Warenpräsentationsmöbels angebracht. D. h., es wird hauptsächlich eine topologische Anordnung in der Weise verfolgt, dass das Warenpräsentationsmöbel zwischen der Kühleinrichtung und der Rücklufteinrichtung angeordnet wird.

Im Übrigen hat es sich bewährt, wenn die Rücklufteinrichtung eine Bauhöhe aufweist, die an der Höhe der abfallenden Kante des Warenpräsentationsmöbels bzw. Warenpräsentationstisches endseitig der Warenauflage bzw. Warenauflageebene angepasst ist. Denn dadurch tritt die gleichsam parallel zur Warenauflageebene über diese hinüberstreichende Kühlluft automatisch in die gegenüberliegende Rücklufteinrichtung ein und kann hier abgesaugt und ggf. in die Kühleinrichtung zurückgeführt werden.

Sowohl das Warenpräsentationsmöbel als auch die Kühleinrichtung sowie schließlich die optionale Rücklufteinrichtung sind vorteilhaft jeweils verfahrbar ausgebildet. Dadurch können die einzelnen Elemente problemlos an unterschiedlichen Aufstellungsorten aufgebaut werden und/oder mit vorhandenen Elementen, beispielsweise einem vorhandenen Warenpräsentationsmöbel kombiniert werden. Um das Warenpräsentationsmöbel, die Kühleinrichtung und die Rücklufteinrichtung miteinander zu koppeln, können diese jeweils miteinander verbunden werden. Alternativ oder zusätzlich ist es aber auch möglich, eine gemeinsame Verbindungseinrichtung vorzusehen. Diese Verbindungseinrichtung mag zugleich als Rückluftkanal fungieren und führt die von der Rücklufteinrichtung angesaugte Kühlluft in die Kühleinrichtung fußseitig zurück, von wo aus diese erneut gekühlt wird und kopfseitig über die Luftführungseinheit wieder austritt und somit im Kreis geführt wird. Auf diese Weise wird die Luftführung optimiert und der Energieverbrauch insgesamt verringert.

Ferner ist es möglich, wenn die Kühleinrichtung zwischen zwei Warenpräsentationsmöbeln angeordnet wird. Endseitig der Warenpräsentationsmöbel bzw. Warenpräsentationstische können jeweils Rücklufteinrichtungen realisiert werden, so dass eine insgesamt spiegelsymmetrische Ausgestaltung im Vergleich zur auf der Spiegelachse angeordneten Kühleinrichtung vorliegt.

Die Kühleinrichtung ist in ihrem Innern mit einem üblichen Wärmetauscher ausgerüstet und mag zusätzlich einen Kondensator sowie eine Luftbefeuchtungseinheit aufweisen. Ferner kann auch eine Aromatisierungseinheit realisiert werden, um die aus der Luftführungseinheit austretende Kühlluft zugleich mit Duftstoffen gezielt anzureichern. Der insgesamt modulare Aufbau ermöglicht es schließlich, dass das Warenpräsentationsmöbel, die Kühleinrichtung und/oder die optionale Rücklufteinrichtung ganz oder teilweise in einem kastenartigen Gehäuse aus beispielsweise Holzplatten, Kunststoffplatten, Metallplatten oder Kombinationen aufgenommen werden können.

Im Ergebnis wird ein Aggregat aus Warenpräsentationsmöbel und Kühleinrichtung zur Verfügung gestellt, welches auf aufwendige Gehäuse und Wandgestaltungen verzichtet und insbesondere bereits vorhandene Warenpräsentationsmöbel mit einbezieht. Hierdurch können auf dem Warenpräsentationsmöbel abgelegte Waren, beispielsweise Obst und Gemüse, problemlos temperiert und gekühlt werden. Gleichzeitig ist eine optionale Befeuchtung und Aromatisierung der Kühlluft möglich.

Die modulare Bauweise erlaubt es zum ersten Mal, bisher ungekühlte Warenpräsentationsmöbel und die darauf befindlichen Produkte zu temperieren oder zu kühlen. Gleichzeitig lässt sich das erfindungsgemäße Aggregat flexibel an unterschiedliche räumliche Gegebenheiten anpassen. Hierin sind die wesentlichen Vorteile zu sehen.

Im Folgenden wird die Erfindung anhand einer lediglich ein Ausführungsbeispiel darstellenden Zeichnung näher erläutert; es zeigen:
- Fig. 1: das erfindungsgemäße Aggregat schematisch,
- Fig. 2: eine erste Ausführungsform perspektivisch und
- Fig. 3: eine abgewandelte Ausgestaltung in Seitenansicht.

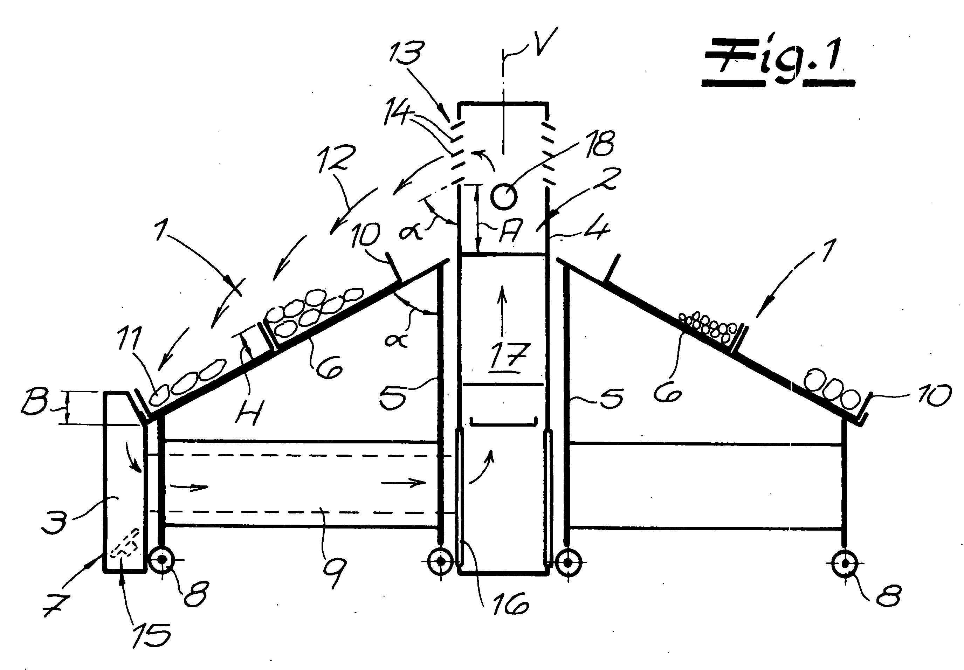

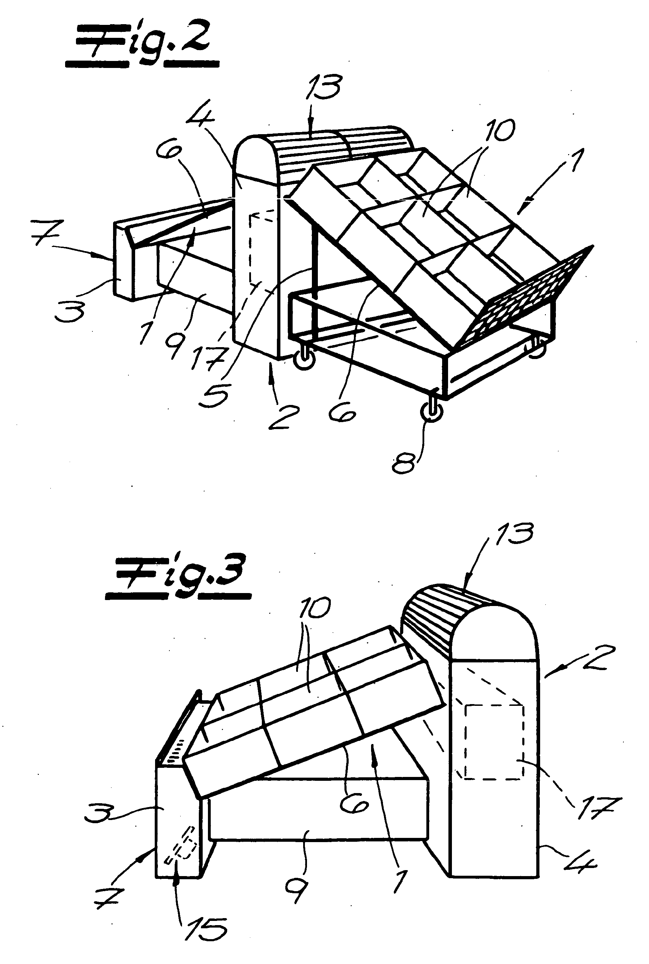

In den Figuren ist ein Aggregat aus wenigstens einem Warenpräsentationsmöbel 1 und einer Kühleinrichtung 2 dargestellt. Man erkennt, dass bei der Variante nach den Fig. 1 und 2 zwei Warenpräsentationsmöbel 1 mit einer Kühleinrichtung 2 kombiniert werden, während das Beispiel nach der Fig. 3 die Kombination eines Warenpräsentationsmöbels 1 mit lediglich einer Kühleinrichtung 2 zeigt. Zum grundsätzlichen Aufbau gehört noch eine optionale Rücklufteinrichtung 3, die bei dem in den Fig. 1 und 2 rechten Warenpräsentationsmöbel 1 nicht realisiert ist, dort aber selbstverständlich vorgesehen werden kann.

Im Rahmen der Erfindung ist nun von besonderer Bedeutung, dass das Warenpräsentationsmöbel 1 und die Kühleinrichtung 2 miteinander verbunden sind und über jeweils ein eigenes Gestell 5, 6 respektive 4 verfügen. Auch die Rücklufteinrichtung 3 ist selbst modular aufgebaut und mit einem eigenen Gestell 7 ausgerüstet. Zusätzlich mögen das Warenpräsentationsmöbel 1, die Kühleinrichtung 2 und die Rücklufteinrichtung 3 ganz oder teilweise mit einem Gehäuse versehen werden. Um den modularen Charakter noch zu steigern, sind das Warenpräsentationsmöbel 1, die Kühleinrichtung 2 und die Rücklufteinrichtung 3 jeweils verfahrbar ausgebildet oder sind nur einzelne der vorerwähnten Elemente 1, 2, 3 mit einer Verfahreinrichtung, beispielsweise angedeuteten Rollen 8 versehen.

Das Warenpräsentationsmöbel 1, die Kühleinrichtung 2 und die Rücklufteinrichtung 3 sind untereinander verbunden, und zwar im Rahmen des Beispiels mit Hilfe einer gemeinsamen Verbindungseinrichtung 9. Bei dieser Verbindungseinrichtung 9 handelt es sich um einen Rückluftkanal bzw. einen hohlen Unterzug, welcher die Rücklufteinrichtung 3 mit der Kühleinrichtung 2 verbindet, so dass dazwischen das Warenpräsentationsmöbel 1 gehalten wird. Selbstverständlich kann das Warenpräsentationsmöbel 1 zusätzlich oder alternativ auch einerseits an die Kühleinrichtung 2 und andererseits an die Rücklufteinrichtung 3 angeschlossen werden. So ist das in den Fig. 1 und 2 rechte Warenpräsentationsmöbel 1 jeweils lediglich mit der Kühleinrichtung 2 verbunden. Insgesamt versteht es sich, dass die einzelnen Elemente 1, 2, 3 des dargestellten Aggregates, d. h. das Warenpräsentationsmöbel 1, die Kühleinrichtung 2 und die Rücklufteinrichtung 3 lösbar miteinander verbunden werden können.

Der grundsätzliche Aufbau ist so gestaltet, dass das Warenpräsentationsmöbel 1 zwischen der Kühleinrichtung 2 und der Rücklufteinrichtung 3 angeordnet ist. Außerdem kann eine Kühleinrichtung 2 mit zwei beidseitigen Warenpräsentationsmöbeln 1 und zwei beidseitigen Rücklufteinrichtungen 3 kombiniert werden, wie dies die Fig. 1 andeutet. In diesem Fall ist eine gleichsam spiegelsymmetrische Ausgestaltung realisiert, bei welcher die Kühleinrichtung 2 die Spiegelachse darstellt bzw. sich auf der Spiegelachse befindet. Das ist selbstverständlich nicht zwingend.

Bei dem Warenpräsentationsmöbel 1 handelt es sich nicht einschränkend um einen Warenpräsentationstisch, der sich im Detail aus einem Gestell 5 und einer schräg gestellten Warenauflage 6 zusammensetzt. Die Warenauflage 6 definiert eine Warenauflageebene, auf der angedeutete Behälter 10 mit Waren 11 aufgenommen werden. Hierbei mag es sich um Obst und/oder Gemüse handeln. Selbstverständlich kann die Warenauflage 6 auch horizontal angeordnet sein.

Man erkennt, dass die Warenauflage 6 im Ausführungsbeispiel gegenüber der im Wesentlichen als senkrechter Turm ausgebildeten Kühleinrichtung 2 schräg gestellt ist. Im Rahmen des Ausführungsbeispiels und nicht einschränkend wird ein Winkel α der Schrägstellung von ca. 60 ° beobachtet, wobei selbstverständlich auch andere Winkel α denkbar sind und umfasst werden, insbesondere dann, wenn die Warenauflage 6 gegenüber dem Gestell 5 verstellt werden kann, und zwar ähnlich wie in der EP 1 493 363 A1 beschrieben, auf die ausdrücklich Bezug genommen wird.

Die Warenauflage 6 bzw. das Warenpräsentationsmöbel 1 ist schräg abfallend - beginnend von der Kühleinrichtung 2 - bis hin zur Rücklufteinrichtung 3 geneigt angeordnet. Dem zugehörigen Winkel α dieser Schrägstellung folgt die Führung der aus der Kühleinrichtung 2 austretenden Kühlluft 12, wie die Fig. 1 deutlich macht. Tatsächlich streicht die Kühlluft 12 in etwa parallel über die Warenauflage 6, damit die dort platzierten Waren 11 gekühlt respektive temperiert werden. Um diese Führung der Kühlluft 12 zu erreichen, verlässt die Kühlluft 12 die Kühleinrichtung 2 kopfseitig durch eine dort realisierte Luftführungseinheit 13. Bei dieser Luftführungseinheit 13 handelt es sich im Ausführungsbeispiel und nicht einschränkend um ein Lamellengitter 13, welches eine Vielzahl von schräg gestellten Lamellen 14 aufweist.

Die Lamellen 14 sind gegenüber der im Wesentlichen senkrecht angeordneten Kühleinrichtung 2 schräg gestellt, wobei an dieser Stelle ein vergleichbarer Winkel α der Schrägstellung beobachtet wird, wie für die Warenauflage 6 gegenüber der Kühleinrichtung 2. Dadurch wird im Wesentlichen erreicht, dass die Kühlluft 12 parallel zur Warenauflage 6 strömt.

Die Kühleinrichtung 2 und die Rücklufteinrichtung 3 sind an jeweils gegenüberliegenden Seiten des Warenpräsentationsmöbels 1 angeordnet. Auf diese Weise strömt die Kühlluft 12 von der Kühleinrichtung 2 parallel zur Warenauflage 6 automatisch in Richtung auf die Rücklufteinrichtung 3, welche an der abfallenden Kante des Warenpräsentationstisches bzw. des Warenpräsentationsmöbels 1 platziert ist.

Die Rücklufteinrichtung 3 verfügt insgesamt über eine Bauhöhe, die der Höhe der abfallenden Kante des Warenpräsentationstisches bzw. Warenpräsentationsmöbels 1 entspricht, so dass die Kühlluft 12 endseitig der Warenauflage 6 unmittelbar in die Rücklufteinrichtung 3 eintreten kann. Unterstützend wirken an dieser Stelle in der Rücklufteinrichtung 3 angeordnete Lüfter 15, welche die Kühlluft 12 in die Rücklufteinrichtung 3 einsaugen.

Ausgangsseitig der Lüfter 15 schließt sich an die Rücklufteinrichtung 3 die Verbindungseinrichtung bzw. der Rückluftkanal 9 an, so dass die in die Rücklufteinrichtung 3 eintretende Kühlluft 12 unmittelbar in die Verbindungseinrichtung 9 entsprechend der Pfeildarstellung in Fig. 1 umgelenkt wird. Von dort aus wird die Kühlluft 12 mit Hilfe eines eingangsseitigen Filters 16 in die Kühleinrichtung 2 eingeblasen, dann nach oben gerichtet umgelenkt und mit Hilfe eines Wärmetauschers 17 - falls erforderlich - gekühlt.

Nach Passieren des Wärmetauschers 17 wird die Kühlluft 12 ggf. mit feinverteiltem Wasser befeuchtet, welches von einer Befeuchtungseinheit 18 im Bereich der Luftführungseinheit 13 zur Verfügung gestellt wird. An Stelle der Befeuchtungseinheit 18 mag auch eine Aromatisierungseinheit an dieser Stelle vorgesehen sein, um der Kühlluft 12 gezielt Duftstoffe zuzuführen. Selbstverständlich kann die Aromatisierungseinheit auch mit der Befeuchtungseinheit 18 kombiniert werden. Im Anschluss hieran wird die Kühlluft 12 in der Luftführungseinheit 13 umgelenkt und tritt erneut aus der Kühleinrichtung 2 aus, um die Waren 11 zu kühlen bzw. zu temperieren.

Im Rahmen des Ausführungsbeispiels nach den Fig. 1 und 2 ist das Warenpräsentationsmöbel 1 als Gittergestell mit in ihrer Schrägstellung einstellbarer Warenauflage 6 ausgebildet. Alternativ hierzu kann aber auch ein kastenartiges Gehäuse entsprechend der Fig. 3 realisiert werden. Ebenso mögen die Kühleinrichtung 2 und die Rücklufteinrichtung 3 über ein kastenartiges Gehäuse verfügen, das beispielsweise aus Holz-/Kunststoffplatten hergestellt ist. Dabei sind das Warenpräsentationsmöbel 1, die Kühleinrichtung 2 und die Rücklufteinrichtung 3 jeweils von ihrer Länge her aneinander angepasst.

Bei der Variante nach den Fig. 2 und 3 erkennt man schließlich, dass die Luftführungseinheit 13 halbzylindrisch ausgebildet ist, wobei die Lamellen 14 jeweils radial angeordnet sind und jeweils gegenüber einer Vertikalen V den beschriebenen Winkel α der Schrägstellung aufweisen. Schließlich mögen ein Abstand A der Luftführungseinheit 13 wie ein Überstand B der Rücklufteinrichtung 3 über die benachbarte Warenauflage 6 an eine Höhe H der Aufnahmebehälter 10 für die Waren 11 angepasst sein, so dass auf diese Weise die Kühleinrichtung 2 und die Rücklufteinrichtung 3 gleichsam eine nach oben offene Wanne für die auf der Warenauflage 6 platzierten Behälter 10 respektive die darin angeordneten Waren 11 formen.

## Patentansprüche

1. Aggregat aus Warenpräsentationsmöbel (1) und hiermit verbundener Kühleinrichtung (2), wobei
- die Kühleinrichtung (2) kopfseitig mit einer Luftführungseinheit (13) ausgerüstet und zusätzlich eine Rücklufteinrichtung (3) vorgesehen ist, wobei sich ferner
- das Warenpräsentationsmöbel (1) aus einem Gestell (5) und einer Warenauflage (6) zusammensetzt sowie
- die Warenauflage (6) gegenüber der im Wesentlichen als senkrechter Turm ausgebildeten Kühleinrichtung (2) schräggestellt ist, und wobei
- die Luftführungseinheit (13) die Warenauflage (6) des Warenpräsentationsmöbels (1) überragt, so dass
- die aus der Luftführungseinheit (13) austretende Kühlluft (12) in etwa parallel über dort platzierte Waren (11) zu deren Temperierung streicht,
**dadurch gekennzeichnet,dass**
- das Warenpräsentationsmöbel (1) und die hiermit verbundene Kühleinrichtung (2) jeweils modular mit zumindest eigenem Gestell (5, 6; 4) ausgebildet sind, wobei
- zusätzlich die Rücklufteinrichtung (3) selbst modular mit zumindest eigenem Gestell (7) ausgeführt ist.

2. Aggregat nach Anspruch 1, **dadurch gekennzeichnet, dass** das Warenpräsentationsmöbel (1) und die Kühleinrichtung (2) mit einem Gehäuse ausgebildet sind.

3. Aggregat nach Anspruch 1 oder 2, **dadurch gekennzeichnet, dass** das Warenpräsentationsmöbel (1) und/oder die Kühleinrichtung (2) verfahrbar ausgebildet sind.

4. Aggregat nach einem der Ansprüche 1 bis 3, **dadurch gekennzeichnet, dass** die Rücklufteinrichtung (3) mit einem Gehäuse sowie optional verfahrbar ausgebildet ist.

5. Aggregat nach einem der Ansprüche 1 bis 4, **dadurch gekennzeichnet, dass** das Warenpräsentationsmöbel (1) und/oder die Kühleinrichtung (2) und/oder die Rücklufteinrichtung (3) jeweils miteinander verbunden sind und/oder eine gemeinsame Verbindungseinrichtung (9) vorgesehen ist.

6. Aggregat nach einem der Ansprüche 1 bis 5, **dadurch gekennzeichnet, dass** die Luftführungseinheit (13) als Lamellengitter (13) ausgebildet ist.

7. Aggregat nach einem der Ansprüche 1 bis 6, **dadurch gekennzeichnet, dass** das Warenpräsentationsmöbel (1) zwischen der Kühleinrichtung (2) und der Rücklufteinrichtung (3) angeordnet ist.

8. Aggregat nach einem der Ansprüche 1 bis 7, **dadurch gekennzeichnet, dass** das Warenpräsentationsmöbel (1) die Kühleinrichtung (2) und/oder die Rücklufteinrichtung (3) ein kastenartiges Gehäuse aus beispielsweise Holz-/Kunststoffplatten aufweist.

## Claims

1. A system of a product display furnishing unit (1) and a refrigeration unit (2) connected to it, wherein
- the refrigeration unit (2) is furnished at the head end with an air supply unit (13) and a return air unit (3), wherein also
- the product display furnishing unit (1) is assembled from a frame (5) and a product display (6) as well as
- the product display (6) being positioned at an inclination with respect to the refrigeration unit (2), which is designed essentially as a vertical tower, and wherein
- the air supply unit (13) protrudes beyond the product display (6) of the product display furnishing unit (1), so that
- the cool air (12) emerging from the air supply unit (13) passes approximately parallel over the product (11) placed there for thermal regulation of same,
**characterized in that**
- the product display furnishing unit (1) and the refrigeration unit (2) connected to it each have a modular design with at least their own frame (5, 6; 4), wherein
- the return air unit (3) also has a modular design itself with at least its own frame (7).

2. The system according to Claim 1, **characterized in that** the product display furnishing unit (1) and the refrigeration unit (2) are designed with a housing.

3. The system according to Claim 1 or 2, **characterized in that** the product display furnishing unit (1) and/or the refrigeration unit (2) is/are designed to be portable.

4. The system according to any one of Claims 1 to 3, **characterized in that** the return air unit (3) is designed with a housing and is optionally portable.

5. The system according to any one of Claims 1 to 4, **characterized in that** the product display furnishing unit (1) and/or the refrigeration unit (2) and/or the return air unit (3) is/are connected to one another and/or a common connecting unit (9) is provided.

6. The system according to any one of Claims 1 to 5, **characterized in that** the air supply unit (13) is designed as a louver grid (13).

7. The system according to any one of Claims 1 to 6, **characterized in that** the product display furnishing unit (1) is arranged between the refrigeration unit (2) and the return air unit (3).

8. The system according to any one of Claims 1 to 7, **characterized in that** the product display furnishing unit (1), the refrigeration unit (2) and/or the return air unit (3) has/have a box-type housing of wood/plastic panels, for example.

## Revendications

1. Groupe formé d'un présentoir de produits (1) et d'un système réfrigérant (2) raccordé sur ce dernier,
- côté tête, le système réfrigérant (2) étant équipé d'une unité de guidage de l'air (13) et un système d'air de retour (3) étant prévu en supplément, par ailleurs,
- le présentoir de produit (1) se composant d'un châssis (5) et d'une tablette pour produits (6) et
- la tablette pour produits (6) étant inclinée par rapport au système réfrigérant (2) sensiblement conçu en tant que tour verticale et
- l'unité de guidage d'air (13) saillant par-dessus la tablette pour produits (6) du présentoir de produits (1) de sorte que
- l'air de refroidissement (12) sortant de l'unité de guidage d'air (13) effleure les produits (11) qui sont placés là approximativement à la parallèle pour les tempérer,
**caractérisé en ce que**
- le présentoir de produits (1) et le système réfrigérant (2) raccordé sur ce dernier sont chacun réalisés de manière modulaire avec au moins un châssis (5, 6 ; 4),
- en supplément, le système d'air de retour (3) étant lui-même conçu de manière modulaire, avec au moins son propre châssis (7).

2. Groupe selon la revendication 1, **caractérisé en ce que** le présentoir de produits (1) et le système réfrigérant (2) sont conçus avec une carcasse.

3. Groupe selon la revendication 1 ou la revendication 2, **caractérisé en ce que** le présentoir de produits (1) et/ou le système réfrigérant (2) sont conçus de manière déplaçable.

4. Groupe selon l'une quelconque des revendications 1 à 3, **caractérisé en ce que** le système d'air de retour (3) est réalisé avec une carcasse et en option de manière déplaçable.

5. Groupe selon l'une quelconque des revendications 1 à 4, **caractérisé en ce que** le présentoir de produits (1) et/ou le système réfrigérant (2) et/ou le système d'air de retour (3) sont raccordés chacun l'un à l'autre et/ou en ce qu'il est prévu un système de raccordement (9) commun.

6. Groupe selon l'une quelconque des revendications 1 à 5, **caractérisé en ce que** l'unité de guidage d'air (13) est conçue sous forme d'un treillage à lamelles (13).

7. Groupe selon l'une quelconque des revendications 1 à 6, **caractérisé en ce que** le présentoir de produits (1) est disposé entre le système réfrigérant (2) et le système d'air de retour (3).

8. Groupe selon l'une quelconque des revendications 1 à 7, **caractérisé en ce que** le présentoir de produits (1), le système réfrigérant (2) et/ou le système d'air de retour (3) comporte une carcasse de type caisson en panneaux de bois ou en matière plastique, par exemple.
